# EUROPEAN PATENT APPLICATION

(11) **EP 4 437 986 A1**
(43) Date of publication of application: **02.10.2024**
(21) Application number: 22945534.0
(22) Date of filing: 09.10.2022
(51) Int. Cl.: A61B 18/12, A61B 18/14, A61B 18/00

(54) **ELECTROPHYSIOLOGICAL CATHETER**

(30) Priority: 06.06.2022 CN 202210629751
(71) Applicant: Insight Medtech Co., Ltd, Shenzhen, Guangdong 518101 (CN)
(72) Inventor: WEI, Shibo, Shenzhen, Guangdong 518101 (CN); YANG, Zhe, Shenzhen, Guangdong 518101 (CN); LUO, Cihua, Shenzhen, Guangdong 518101 (CN)
(74) Representative: KIPA AB
(86) International application number: PCT/CN2022/123977
(87) International publication number: WO 2023/236402

(57) **Abstract**

The present disclosure relates to an electrophysiology catheter which includes an inner tube and an outer tube connected in a sleeving mode. The inner tube and the outer tube are movably relative to each other, and the inner tube and the outer tube are connected with each other by an electrode assembly. The electrode assembly includes a plurality groups of base body groups and electrodes. The plurality groups of base body groups are arranged in a circumferential direction of the inner tube. The base body group connects the inner tube with the outer tube and includes two or more base bodies distributed at intervals in the circumferential direction of the inner tube The base body is deformable and at least includes a first segment and a second segment connected with each other. The first segment and the second segment have a first connecting point. The first connecting points of a same base body group are in contact connection with each other to form a junction. The electrodes are at least disposed at the junctions. The electrophysiology catheter provided in the present disclosure can improve the ablation efficiency while improving the stability of the electrodes at the ablation positions and obtain a better ablation effect.

## Description

### CROSS-REFERENCE TO RELATED APPLICATIONS

The present disclosure claims priority to Chinese Patent Application No. 202210629751.4 filed with the China National Intellectual Property Administration on June 06, 2022, entitled electrophysiology catheter, the contents of which are all incorporated herein by reference in their entireties.

### FIELD OF THE INVENTION

The present disclosure relates to the field of medical instrument technology, particularly to electrophysiology catheters.

### DESCRIPTION OF THE RELATED ART

With the increasing number of patients in the heart rate market, the continuous development of medical technology, and the advancement of minimally invasive intervention instruments, the field of electrophysiology has gradually received more attention. The instruments related to cardiac electrophysiological intervention technology mainly include mapping catheters, ablation catheters, and other electrophysiological intervention devices. The mapping catheters and most of the ablation catheters (radiofrequency ablation and pulsed field ablation) are collectively referred to as electrophysiology catheters. Metal electrodes are required on the catheter as a medium for signal or energy transmission, and the shape and density of the electrodes have a significant impact on the efficiency and effect of the mapping catheters and ablation catheters (radiofrequency ablation and pulsed field ablation). At present, when using pulse ablation technology to treat paroxysmal atrial fibrillation, the electrophysiological catheter used has poor internal support strength and stability, which can easily cause the displacement of electrodes, affecting the attachability and uniformity of electrodes and affecting ablation efficiency and effect.

### TECHNICAL PROBLEM

To solve or partially solve the problems existing in the related art, the present disclosure provides an electrophysiology catheter which can improve the ablation efficiency while improving the stability of the electrodes at the ablation positions and obtain a better ablation effect.

### SUMMARY

The present disclosure provides an electrophysiology catheter which includes an inner tube and an outer tube connected in a sleeving mode. The inner tube is movably relative to the outer tube, and the inner tube and the outer tube are connected with each other by an electrode assembly which includes a plurality groups of base body groups and electrodes.

The plurality groups of base body groups which are arranged in a circumferential direction of the inner tube, wherein the base body group connects the inner tube with the outer tube and includes two or more base bodies distributed at intervals in the circumferential direction of the inner tube, the base body is deformable and at least includes a first segment and a second segment connected with each other, the first segment and the second segment have a first connecting point, the first connecting points of a same base body group are in contact connection with each other to form a junction; and

The electrodes which are at least disposed at the junctions.

In an implementation, the electrode assembly at least has a first state and a second state, and the relative movement between the inner tube and the outer tube can drive the connected electrode assembly to switch between the first state and the second state.

In the first state, the base body is attached to an outer wall of the inner tube in the circumferential direction of the inner tube, and an extension direction of the base body is parallel to an axis direction of the inner tube;

In the second state, the base body bendingly deform to outwardly bulge relative to the inner tube, each adjacent first segments of a same base body group enclose to form a first closed shape and each adjacent second segments enclose to form a second closed shape, and each adjacent base body groups enclose to form a third closed shape.

In an implementation, in the second state, the plurality groups of base body groups circumferentially distributed around the inner tube have a largest diameter, and the junctions are located at one side of a plane where the largest diameter is located away from the outer tube.

In an implementation, the electrodes at least include first electrodes and second electrodes, wherein the first electrodes are located at the junctions, the second electrodes are disposed at the second segments, and the second electrodes are located at the positions with the largest diameter, or the second electrodes are located between the positions with the largest diameter and the junctions.

In an implementation, the first electrodes and the second electrodes are electrode rings in an annular structure, and following the circumferential direction of the inner tube, a circumferential area of the first electrodes is greater than a circumferential area of the second electrodes.

In an implementation, along the axis direction of the inner tube, the first electrodes circumferentially arranged have a first diameter, and the second electrodes circumferentially arranged have a second diameter which is greater than the first diameter.

In an implementation, the electrode assembly includes a plurality of first base seats which are circumferentially arranged on the inner tube and configured to connect the plurality groups of base body groups with the inner tube, and the base bodies of a same base body group are connected to different said first base seats respectively; and/or

The electrode assembly includes a plurality of second base seats which are circumferentially arranged between the inner tube and the outer tube at one side near the outer tube and configured to connect the plurality groups of base body groups with the outer tube, and the base bodies of a same base body group are connected to different said second base seats respectively.

In an implementation, the second base seat and the second segment have a second connecting point at the connecting position, the base body group includes two base bodies, and the second connecting points at the connecting positions of adjacent base body groups with the same second base seat are in contact fit with each.

The electrodes further include third electrodes, and the third electrode is located at the second connecting points which are in contact fit with each other.

In an implementation, the electrode assembly has a third state which is formed by a relative movement between the inner tube and the outer tube on the basis of the second state.

In the third state, the third electrodes are circumferentially arranged in interspaces between the base body groups, the electrode assembly forms an umbrella structure, and along the axis direction of the inner tube the plane defined by the third electrodes is at least between the first electrodes and the second electrodes.

In an implementation, the electrode assembly has a fourth state which is formed by the relative movement between the inner tube and the outer tube on the basis of the second state.

In the fourth state, the third electrodes are circumferentially arranged in the interspaces between the base body groups and at least the first electrodes, the second electrodes and the third electrodes are in a same plane.

In an implementation, the base body includes a support rod and an insulating layer, the support rod is a metal member with deformation ability, and the insulting layer covers a surface of the metal member.

### ADVANTAGES

An electrophysiology catheter includes an inner tube and an outer tube connected in a sleeving mode. The inner tube and the outer tube are movably relative to each other, and the inner tube and the outer tube are connected with each other by an electrode assembly which includes a plurality groups of base body groups and electrodes. The plurality groups of base body groups are arranged in a circumferential direction of the inner tube. The base body group connects the inner tube with the outer tube and includes two or more base bodies distributed at intervals in the circumferential direction of the inner tube. The base body is deformable and at least includes a first segment and a second segment connected with each other. The first segment and the second segment have a first connecting point. The first connecting points of a same base body group are in contact connection with each other to form a junction. The electrodes are at least disposed at the junctions. For the plurality of base bodies of the base body group, comparing the junction, which is formed by connecting the plurality of first connecting points, with the first segment and the second segment, the structural stability and strength at the junction are significantly increased. When the electrode is disposed at the junction, it is beneficial for the electrode at the junction to form a larger surface area as the electrode ablation area so as to improve the performance of the electrode. Besides, with the arrangement of the junctions, when the plurality groups of base body groups are distributed in the circumferential direction of the inner tube, the junctions have higher strength than other parts of the base bodies. Thus, in the case that the electrodes are disposed at these positions, when the electrode assembly is anchored at the pulmonary vein ostium, the junctions can ensure the stable attachment of the electrodes both in the axial direction and in the radial direction, provide stronger support stability for the electrodes, and are not easy to wobble radially, therefore the uniform distribution of the electrodes in the circumferential direction can be maintained, which is beneficial for the ablation. In addition, as the junction is formed by the contact fitting of the plurality of first connecting points, during the relative movement between the inner tube and the outer tube, the base body is easily restricted by the position of the junction with higher strength so that the deformation of the plurality group of base body groups is more controllable, which prevent the electrodes from being susceptible to external interference so as to affect the uniformity and the circumferential arrangement with the same diameter of the electrodes.

It should be understood that the foregoing general description and the following detailed description are illustrative and explanatory only and do not limit the present disclosure.

### BRIEF DESCRIPTION OF THE DRAWINGS

By providing a more detailed description of the exemplary embodiments of the present disclosure in combination with the accompanying drawings, the foregoing and other purposes, features, and advantages of the present disclosure will become more apparent. In the exemplary embodiments of the present disclosure, the same reference numerals usually represent the same component.
Figure 1 shows the structure of an electrode assembly at the head position of an electrophysiology catheter according to an embodiment of the present disclosure;
Figure 2 shows the structure of an electrode assembly at the head position of an electrophysiology catheter in another direction according to an embodiment of the present disclosure;
Figure 3 is the main view of the structure of an electrode assembly without electrodes at the head position of an electrophysiology catheter according to an embodiment of the present disclosure;
Figure 4 is a top view of the electrophysiology catheter in Figure 1;
Figure 5 shows the structure of the second type of electrode assembly at the head position of an electrophysiology catheter according to an embodiment of the present disclosure;
Figure 6 is the main view of the electrophysiology catheter in Figure 5;
Figure 7 is a top view of the electrophysiology catheter in Figure 5;
Figure 8 shows the structure of the third type of electrode assembly at the head position of an electrophysiology catheter according to an embodiment of the present disclosure;
Figure 9 shows the structure of the fourth type of electrode assembly at the head position of an electrophysiology catheter according to an embodiment of the present disclosure;
Figure 10 is a top view of the electrophysiology catheter in Figure 9;
Figure 11 shows the structure of the fifth type of electrode assembly at the head position of an electrophysiology catheter according to an embodiment of the present disclosure;
Figure 12 is the main view of Figure 11;
Figure 13 is the top view of Figure 11.

### DETAILED DESCRIPTION OF THE EMBODIMENTS

Some embodiments of the present disclosure will be described in more detail below with reference to the accompanying drawings. Although some embodiments of the present disclosure are shown in the accompanying drawings, it should be understood that the present disclosure can be implemented in various forms and should not be limited by the embodiments described here. On the contrary, these embodiments are provided to make the present disclosure more thorough and complete, and to fully convey the scope of the present disclosure to ones skilled in the art.

The terms used in the present disclosure are for the purpose of describing specific embodiments only and are not intended to be limit of the present disclosure. The singular forms of "a", "said", and "the" used in the present disclosure and the accompanying claims are intended to include the plural forms as well, unless the context clearly indicates other meanings. It should also be understood that the term "and/or" used herein includes any or all possible combinations of one or more related listed items.

It should be understood that although the terms "first," "second," "third," etc. may be used herein to describe various information, the information should not be limited to these terms. These terms are only used to distinguish the same type of information from each other. For example, without departing from the scope of the present disclosure, first information can also be referred to as second information, and similarly, second information may also be referred to as first information. Therefore, a feature defines as "first" or "second" can explicitly or implicitly include one or more of that feature. In the description of the present disclosure, "a plurality of" means two or more, unless otherwise specified.

In surgery using electrophysiological therapy, using a catheter to transfer energy and perform tissue ablation or mapping is a common method. The catheter is provided with electrodes serving as a medium for signal or energy transfer so as to achieve the goal. It should be understandable that instruments related to the electrophysiology catheters used for cardiac electrophysiological intervention technology mainly include mapping catheters, ablation catheters and other electrophysiology intervention devices, which are collectively referred to as electrophysiology catheters and are not specifically distinguished herein. For example, when pulsed ablation technology is adopted to treat paroxysmal atrial fibrillation, the electrophysiology catheter needs to enter through the blood vessel, and the head of the catheter needs to be provided with electrodes to unfold into a corresponding shape and anchor at the pulmonary vein ostium. Therefore, for electrodes, their shape, arrangement, density and attachability to the tissue all have a great impact on the efficiency and effect of the use of the electrophysiology catheter.

Aiming at the above problem, an embodiment of the present disclosure provides an electrophysiology catheter which can improve the ablation efficiency while improving the stability of the electrodes at the ablation positions.

The technical schemes according to some embodiment of the present disclosure will be described in detail in combination with the attached drawings.

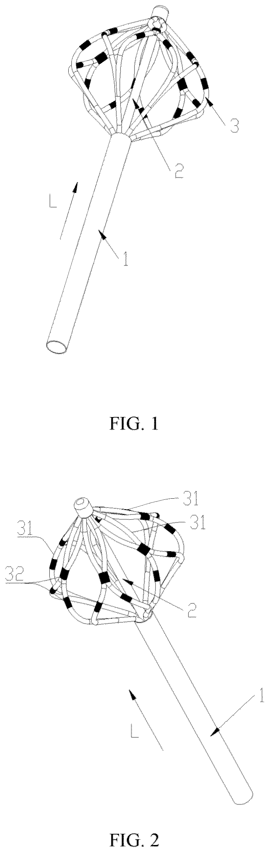

FIG. 1 shows the structure of an electrode assembly at the head position of an electrophysiology catheter according to an embodiment of the present disclosure.

Referring to Figure 1, according to an embodiment of the present disclosure, the electrophysiology catheter includes an inner tube 2 and an outer tube 1 connected in a sleeving mode. The inner tube 2 and the outer tube 1 are movably relative to each other. The inner tube 2 at least partially protrudes from the outer tube 1, and the protruding portion is provided with an electrode assembly 3 which connects the inner tube 2 with the outer tube 1. The relative movement between the inner tube 2 and the outer tube 1 deforms the electrode assembly 3, which is beneficial for the electrode assembly 3 to not obstruct its insertion into the blood vessel and to be able to be attached against the treatment sites to perform the ablation during treatment.

The electrophysiology catheter has a head end and a tail end in the extension direction thereof. The electrode assembly 3 is located at the head end, and the tail end is provided with an adjusting part. The adjusting part connects the sleeved inner tube 2 and the outer tube 1, and can control and drive the inner tube 2 to move relative to the outer tube 1 in the axis direction L (extension direction) of the catheter. Optionally, the adjusting part includes a button and a sliding component slidably connecting the inner tube 2 with the outer tube 1. By pushing the button, the sliding component can be driven to generate the matched movement in the axis direction, and then drives the inner tube 2 to move relative to the outer tube 1 in the axis direction, thereby controlling the corresponding deformation of the electrode assembly 3 at the head end. The adjusting part can alternatively be configured to be other matching structure that can control the relative axial movement of the inner tube 2 and the outer tube 1, which is not specifically limited herein.

It should be understandable that the axes of the inner tube 2 and the outer tube 1 coincide with each other. In order to facilitate entry into the position that needs to be anchored along the blood vessel, the inner tube 2 and the outer tube 1 are both configured to be deformable hoses, the specific materials of which are not described in detail herein.

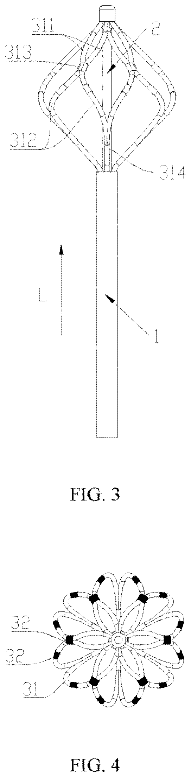

Refer to Figures 2, 3 and 4, the electrode assembly 3 includes a plurality groups of base body groups 31 and electrodes 32. The plurality groups of base body groups 31 are arranged in the circumferential direction of the inner tube 2. The base body groups 31 connect the inner tube 2 with the outer tube 1. The base body group 31 includes at least two base bodies distributed at intervals in the circumferential direction of the inner tube 2. The base body is deformable. The base body at least includes a first segment 311 and a second segment 312 connected with each other. The first segment 311 and the second segment 312 have a first connecting point, and the first connecting points of the same base body group 31 are in contact connection with each other to form a junction 313. The electrodes 32 are at least disposed at the junctions 313.

Specifically, for the inner tube 2 and the outer tube 1 connected in a sleeving mode, the inner tube 2 at the head end at least partially protrudes from the outer tube 1, and the electrode assembly 3 is located at the protruding part. In addition, for the electrode assembly 3, the base body groups 31 are the support structure for arranging the electrodes 32, and the base body groups 31 are arranged in the circumferential direction of the inner tube 2 so that the electrodes 32 disposed on the base body groups 31 can be at least arranged circumferentially on the inner tube 2 and the electrodes 32 can therefore simultaneously perform the ablation in the circumferential direction without requiring to rotate the electrophysiology catheter circumferentially to rotate the electrodes to achieve ablation, which can improve the ablation efficiency.

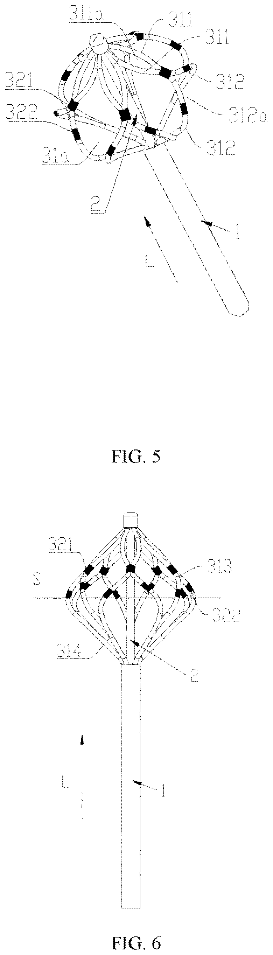

In some implementations, the base body groups 31 are uniformly arranged in the circumferential direction of the inner tube 2. The junctions 313 of the plurality groups of base body groups 31 are uniformly arranged in the circumferential direction so that the electrodes 32 arranged on the base body groups 31 can be uniformly distributed in the circumferential direction. When using the electrodes 32 to perform the ablation, uniform ablation to the corresponding positions by the electrodes 32 distributed circumferentially can be achieved and the ablation effect can be therefore improved. Optionally, the number of the base body groups 31 distributed in the circumferential direction can be adjusted adaptively according to the requirements to the arrangement density of the electrodes. For example, the number of the base body groups 31 can be set to six as shown in Figure 2, or be set to five as shown in Figure 5, which is not specifically limited herein.

Optionally, the junctions 313 of the plurality groups of base body groups 31 are located in the same plane. The plane which is defined by the electrodes 32 disposed at the junctions 313 and the axis direction L of the inner tube 2 may be perpendicular to each other. Or, the two may intersect to form a certain angle which can be adjusted according to different ablation scenarios, which is not specifically limited herein.

The base body group 31 includes two or more base bodies distributed sequentially in the circumferential direction of the inner tube 2. The base body is deformable. The two ends of the base body in the length direction are respectively connected with the outer wall of the inner tube 2 and the inner wall of the outer tube 1. The base body can be a metal member or a non-metal member with elastic deformation ability, and the base body can be obtained by cutting a tubular structure, which can ensure the base body has high support stability and a reduced weight as well, which is beneficial for the convenience of passing through the human body.

Optionally, the base body includes a support rod and an insulating layer. The support rod is a metal member with deformation ability and is a tubular structure. The insulting layer covers the surface of the metal member. The support rod of metal has high support strength and support stability. The insulation layer is used to block the conductive properties of the support rod, and insulate the electrode on the surface of the insulation layer from the support rod. Thus, when the electrode is used, the support rod will not function as a conductive medium to divide the pulse acting on the electrode 32 to result in a decrease in the intensity of the field, so that the using effect of the electrode 32 can be ensured.

It should be understandable that the provided insulation layer may be of a material with good insulation, elasticity and resistance to folding, such as PET, PVC or TPU, and the insulation layer may be an insulating coating applied to the surface of the support rod.

For the two or more base bodies of the base body group 31, the base body specifically includes the first segment 311 and the second segment 312 connected with each. The first segment 311 and the second segment 312 have a connecting point at their connecting position. The first connecting points of the same base body group 31 are in contact fit with each other to form a junction 313. The ends of the first segment 311 and the second segment 312 away from the first connecting point are used to connect the inner tube 2 and the outer tube 1 respectively. For the plurality of base bodies of the base body group 31, comparing the junction 313, which is formed by connecting the plurality of first connecting points, with the first segment 311 and the second segment 312, the structural stability and strength at the junction 313 are significantly increased. When the electrode 32 is disposed at the junction 313, it is beneficial for the electrode 32 at the junction 313 to form a larger surface area as the electrode ablation area so as to improve the performance of the electrode 32. Besides, with the arrangement of the junctions 313, when the plurality groups of base body groups 21 are distributed in the circumferential direction of the inner tube 2, the junctions 313 have higher strength than other parts of the base bodies. Thus, in the case that the electrodes 32 are disposed at these positions, when the electrode assembly 3 is anchored at the pulmonary vein ostium, the junctions 313 can ensure the stable attachment of the electrodes 32 both in the axial direction and in the radial direction, provide stronger support stability for the electrodes 32, and are not easy to wobble radially, therefore the uniform distribution of the electrodes 32 in the circumferential direction can be maintained, which is beneficial for the ablation. In addition, as the junction 313 is formed by the contact fitting of the plurality of first connecting points, during the relative movement between the inner tube 2 and the outer tube 1, the base body is easily restricted by the position of the junction 313 with higher strength so that the deformation of the plurality group of base body groups 31 is more controllable, which prevent the electrodes 32 from being susceptible to external interference so as to affect the uniformity and the circumferential arrangement with the same diameter of the electrodes 32.

Taking the case that the base body group has two base bodies as an example, the electrode assembly 3 according to the present disclosure will be described in detail.

In an embodiment, referring to Figures 5, 6 and 7, the electrode assembly 3 at least has a first state and a second state. The relative movement between the inner tube 2 and the outer tube 1 can drive the connected electrode assembly 3 to switch between the first state and the second state. In the first state, the base bodies are attached to the outer wall of the inner tube 2 in the circumferential direction of the inner tube 2. The extension directions of the base body are parallel to the axis direction of the inner tube 2. In the second state, the base bodies bendingly deform to outwardly bulge relative to the inner tube 2. Each adjacent first segments 311 of the same base body group 31 enclose to form a first closed shape 311a and each adjacent second segments 311 enclose to form a second closed shape 312a. Each adjacent base body groups enclose to form a third closed shape 31a. By this configuration, the better passing ability of the electrode assembly 3 in the human body in the first state can be ensured while the electrodes 32 can be provided with better support stability by the base body groups 31 in the second state.

Specifically, the first state is the tightened state. In the first state, the base bodies of the plurality groups of base body groups 31 distributed in the circumferential direction of the inner tube are respectively attached to the outer wall of the inner tube 2 so that the largest diameter does not exceed the outer diameter of the outer tube 1 so as to facilitate the passing of the electrode assembly 3 in the human body. In addition, in the tightened state, the first segments 311 and the second segments 312, no matter in the same base body group 31 or between adjacent base body groups, can respectively be closely fitted with each other or distributed at intervals so that more base bodies can be arranged while the tightened effect is achieved. The second state is the expanded state. After the electrode assembly 3 enters the human body in the first state and arrives at the pulmonary vein ostium that needs to be anchored, the electrode assembly 3 can be switched to the second state by controlling the movement of the inner tube 2 relative to the outer tube 1 in the axis direction L. In the second state, the base bodies deform to form a hollow spherical structure between the plurality group of base body groups 31, and the base body groups 31 form the skeleton of the sphere. In the spherical structure, the provided junctions result in the closed shapes are formed between the first segments 311, between the second segments 312 and between adjacent base body groups 31 so that a mesh sphere looking like a mesh shape is formed and the junction 313 is a bond of the plurality of first connecting points. When being a part of each closed shape, it can make the structure of each closed shape more stable. Meanwhile, by disposing the electrode 32 at the junction 313, it is beneficial for the electrode 32 to be better attached against the pulmonary vein and be not easy to cause displacement between the circumferentially arranged electrodes 32 due to radial rotation force, which makes the ablation regions of the electrodes uniform, stable and continuous.

More specifically, for the provided junction 313, its transverse cross-sectional area along the axis direction L of the catheter is greater than the maximum transverse cross-sectional area of any of the base bodies. When the plurality of first connecting points are in contact fit with each other to form the junction 313, the strength of the base bodies can be effectively increased. By disposing the electrode 32 at the junction 313, it is beneficial for the stability of the electrode 32 so that it is not easy to deform when being anchored at the pulmonary vein ostium, and the electrodes 32 can be uniformly arranged.

Optionally, when the plurality of first connecting points are in contact connection with each other to form the junction 313, they can be connected as a whole by welding or other operations. In order to facilitate disposing the electrode 32 at the provided junction 313, the outer surface of the junction 313 transitions smoothly when the plurality of first connecting points are in contact connection with each to form the junction 313. The cross section of the junction can be elliptical or polygonal tubular structure, and the electrode 32 can be wound around the circumference of the junction.

Corresponding to the provided junction 313, a surface radially away from the inner tube 2 is an ablation surface. The ablation surface can be attached to the surface of the pulmonary vein in the second state. The corresponding length of the ablation surface in the circumferential direction is greater than the width of any one of the base bodies in the circumferential direction so as to improve the ablation efficiency.

It should be understandable that for the provided junction 313, its outer wall at least includes the first surfaces opposing each other and the second surfaces. The second surfaces are surfaces facing to adjacent base body groups 31 in the circumferential direction of the catheter. The first surfaces are two opposite surfaces in the radial direction of the catheter, and the first surface at one side away from the inner tube 2 corresponds to the ablation surface. In the first state, the first surface at one side facing to the axis can at least partially press against the outer wall of the inner tube 2. The second surfaces between adjacent base body groups 31 can be in contact with each other or have an interspace. When the electrode assembly 3 is switched from the first state to the second state, the junctions 313 move to the radial direction away from the axial direction L of the catheter, resulting in the deformation of the first segments 311 and the second sections 312 of the base bodies, and the diameter defined by the junctions 313 which are arranged in the circumferential direction increases to make at least part of the ablation surfaces be able to attach to the tissue. The junction 313 can provide the electrode 32 with a stronger and more stable support force and also increase the contact area when the ablation surface is attached against the tissue, which can ensure the effect of the attachment of the ablation surface with the tissue.

When the electrophysiology catheter is anchored at the pulmonary vein ostium in the second state, the base bodies bendingly deform to form a spherical structure in combination. After the base bodies are connected to the inner tube 2 and the outer tube 1, the formation of the joints 313 make the first closed shape 311a, the second closed shape 312a and the third closed shape 31a, etc. formed within and between the base body groups 31 so that the mesh structures with different sizes are formed in the spherical structure. The first closed shapes 311a and the second closed shapes 312a formed by the arranged base body group 31 can be elliptical structures so that the electrodes 32 located at the junctions 313 have high reliability in the radial direction, so as to improve the support stability of the base bodies. The base body groups 31 can form a firm quadrilateral structure by the formation of the junctions 313, which ensures the stability of the electrode 32 in the axial direction. Thus, the entire electrode assembly 3 can form a solid whole to ensure the stability of the relative positions of the electrodes 32 in the second state, and ensure the uniform stability and continuity of the electrode ablation regions.

It should be understandable that in order to enable the base bodies to deform to the corresponding shapes and maintain the corresponding shapes in the desired state when the electrode assembly 3 is switched to a different state, it is necessary to preforming the base bodies before the installation and formation of the electrode assembly 3 so that the base bodies can be easily and quickly switched between the first state and the second state and have better formation stability in different states. In general, in the case that the base body includes the support rod and the insulation layer, the preforming treatment is mainly targeted to the support rod with deformation ability of the base body, then the insulation layer is provided on the support rod. The performed preforming treatment is a commonly used means to form the support rod by heat treatment, which is not illustrated in detail herein.

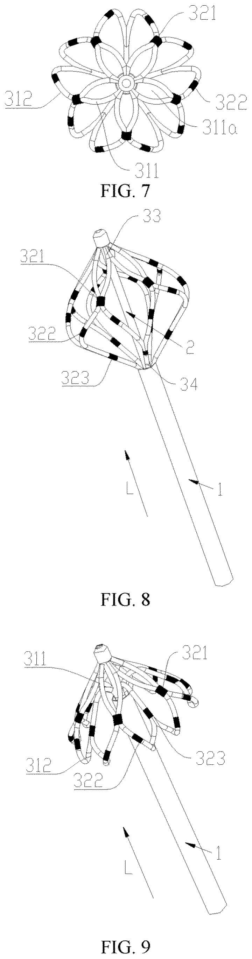

In an embodiment, referring to Figures 6 and 7, the plurality groups of base body groups 31 circumferentially distributed around the inner tube 2 have the largest diameter in the second state. The junctions 313 are located at one side of the plane S where the largest diameter is located away from the outer tube. By this configuration, the junctions 313 can be circumferentially arranged at the upper part of the spherical structure in the axial direction, so that the electrodes 32 can have a better abutment effect and facilitate the circumferentially arranged electrodes 32 to realize the uniform ablation. Optionally, the second closed shape 312a is larger than the first closed shape 311a, which can improve the structural stability at the positions of the joints 313.

As a specific implementation of the present disclosure, continuing with Figures 6 and 7, the electrodes 32 at least includes the first electrodes 321 and the second electrodes 322. The first electrodes 321 are located at the junctions 313. The second electrodes 322 are disposed at the second segments 312, and the second electrodes 322 are located at the positions with the largest diameter. Or, the second electrodes 322 are located between the positions with the largest diameter and the junctions 313. In this structure, with the arrangement of the first electrodes 321 and the second electrodes 322, while the first electrodes 321 and the second electrodes 322 are respectively distributed in their respective circumferential directions, distributing the first electrodes 321 and the second electrodes 322 in the axis direction L of the catheter can increase the ablation area and depth in the axial direction and increase the number of electrodes 32, which results in an increased ablation area for a single ablation of the electrophysiology catheter in both axial and radial directions. The provided second electrodes 322 are uniformly distributed in an annular shape in the electrode assembly 3 and can be used for mapping to achieve the mapping function.

Optionally, the first electrode 321 and the second electrode 322 are electrode rings in an annular structure. Following the circumferential direction of the inner tube 2, the circumferential area of the first electrodes 321 is greater than the circumferential area of the second electrodes 322. By configuring the electrodes 32 in the form of electrode rings, it is easy to install and fix the electrodes to the base bodies. The annular shape of the electrode ring will be adaptively adjusted according to the shape of the cross-section of the base body. After the electrodes are arranged in annular structures at corresponding positions, the plurality of first electrodes 321 are arranged in the circumferential direction of the inner tube 2, and the plurality of second electrodes 322 are arranged in the circumferential direction of the inner tube 2. Moreover, for the annular ring enclosed by the plurality of first electrodes 321 circumferentially arranged, as the first electrodes 321 are located at the junctions 313, the area that each first electrode 321 can expose to the outside is greater than the area that the second electrode 322 can expose to the outside, and the area exposed to the outside can be used as the ablation area. Therefore, for the first electrode 321 arranged in the junction 313, a larger effective area can be arranged in the first electrode 321 as the ablation area so as to compensate for the large interspace between adjacent first electrodes 321 in the circumferential direction.

It should be understandable that, along the axis direction L of the inner tube 2, the first electrodes 321 circumferentially arranged have the first diameter, and the second electrodes 322 circumferentially arranged have the second diameter which is greater than the first diameter. That is, the plurality of first electrodes 321 are arranged in the circumferential direction of the inner tube 2 at the first diameter, the plurality of second electrodes 322 are arranged in the circumferential direction of the inner tube 2 at the second diameter, the first electrodes 321 are located at the junctions 313, and the second electrodes 322 are located at the second segments 312. Making the first electrodes 321 with the larger ablation area be preferentially in contact with the tissue and have a better attachment effect is beneficial to improve the ablation efficiency and effect. Besides, due to the configuration that the second diameter is greater than the first diameter, the second electrodes 322 can be used as the mapping electrodes.

In an embodiment, referring to Figure 8, the electrode assembly 3 includes a plurality of first base seats 33 which are circumferentially arranged on the inner tube 2 and configured to connect the plurality groups of base body groups 31 with the inner tube 2, and the base bodies of the same base body group 31 are connected to different first base seats 33 respectively. And/or, the electrode assembly 3 includes a plurality of second base seats 34 which are circumferentially arranged between the inner tube 2 and the outer tube at one side near the outer tube and configured to connect the plurality groups of base body groups 31 with the outer tube 1, and the base bodies of the same base body group 31 are connected to different second base seats 34 respectively. The first base 33 is detachably connected to the head end of the inner tube 2. The head end of the inner tube 2 is provided with a mounting part. The first base seat 33 connects the mounting part with the base body groups 31. The plurality of first base seats 33 are circumferentially arranged to the mounting part. The second base seat 34 is detachably connected to the inner wall of the outer tube 1 and at least partially protrudes from the outer tube 1. The second base seats 34 are circumferentially arranged on the inner wall of the outer tube 1 to correspond to the corresponding number of the base body groups 31. The base body group 31 includes the first base body and the second base body. The first base body and the second base body between adjacent base body groups are connected to the same base seat so that the closed structures are formed between the first segments 311 and between the second segments 312 of the same base body group and the closed structure is formed between adjacent base body groups 31. With this design of connecting manner, the structural stability of and between the base body groups 31 can be improved, and more electrodes can be arranged, which is beneficial for the uniformity and efficiency of ablation.

Optionally, the second base 34 and the second segment 312 have a second connecting point 314 at the connecting position. The base body group 31 includes two base bodies. The second connecting points 314 at the connecting positions of adjacent base body groups 31 with the same second base seat 34 are in contact fit with each. The electrodes 32 further include the third electrodes 323. The third electrode 323 is located at the second connecting points 314 which are in contact fit with each other. By disposing the third electrode 323 at the second connecting points 314, the ablation area of the third electrode 323 can be increased, meanwhile the structural stability of the third electrode 323 can be improved. The third electrodes 323 can be used as mapping electrodes or as ablation electrodes, which it is not specifically limited herein. Moreover, with the arrangement of the third electrodes 323, the ablation area in the axial direction L of the catheter can be increased.

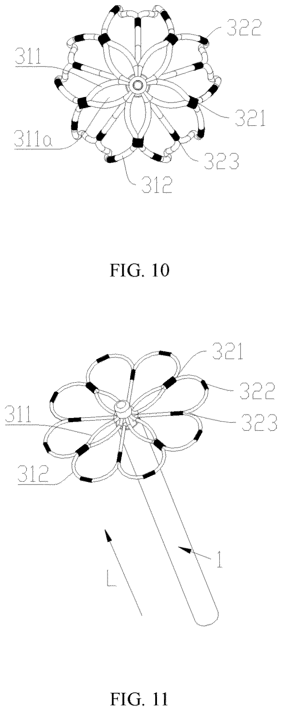

In an embodiment, referring to Figures 9 and 10, the electrode assembly 3 has a third state. The third state is formed by the relative movement between the inner tube 2 and the outer tube 1 close to each other on the basis of the second state. In the third state, the third electrodes 323 are circumferentially arranged in the interspaces between the base body groups 31. The electrode assembly 3 forms an umbrella structure, and along the axis direction L of the inner tube 2, the plane defined by the third electrodes 323 is at least between the first electrodes 321 and the second electrodes 322. With the setting of the third state, in combination with the third electrodes 323 disposed at the second connecting points 314, it is allowed for the electrode assembly 3 to form an umbrella structure in the third state and for the third electrodes 323 located at the second connecting points 314 to enter the interspaces between the base body groups 31 in this state so that the third electrodes 323 can be used as the ablation electrodes and the density of the circumferentially distributed ablation electrodes and the ablation efficiency can be increased when the third electrodes are in cooperation with the first electrodes.

Optionally, in the third state, the first electrodes 321, the second electrodes 322 and the third electrodes 323 form ring structures sleeved with different diameters, when viewing from the axis direction L of the inner tube 2. The diameter of the annular third electrodes 323 is the smallest and the diameter of the second electrodes 322 is the largest. The first electrodes 321, the second electrodes 322 and the third electrodes 323 are arranged in a staggered manner so that the arrangement of the electrodes 32 is more uniform and the ablation efficiency can be improved.

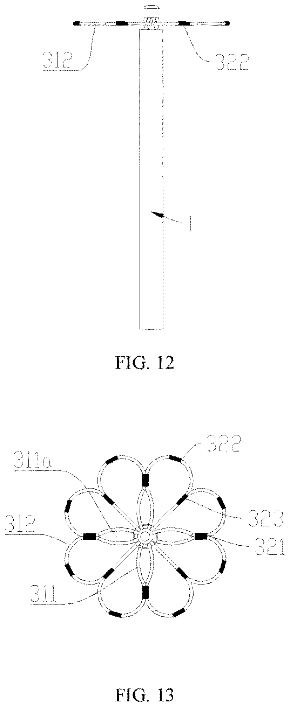

In an optional embodiment, referring to Figures 11 to 13, the electrode assembly 3 has a fourth state. The fourth state is formed by the relative movement between the inner tube 2 and the outer tube 1 on the basis of the second state. In the fourth state, the third electrodes 323 are circumferentially arranged in the interspaces between the base body groups 31. At least the first electrodes 321, the second electrodes 322 and the third electrodes 323 are in a same plane. In this state, the arrangement of different electrodes being at the same plane allows all the electrodes to be used to be attached to the tissue, thus the ablation area is increased, which is beneficial for improving the ablation efficiency and effect. In this state, the third electrodes 323 between the base body groups 31 can be arranged in the circumferential direction of the inner tube 2 at the same diameter with the first electrodes, and the first electrodes 321 and the third electrodes 323 are staggeredly arranged in the circumferential direction of the inner tube 2. Optionally, the plane defined by the first electrodes 321, the second electrodes 322, and the third electrodes 323 can be perpendicular to the axis direction L of the inner tube 2, or can intersect the axis of the inner tube 2 at a certain angle, and the diameter of the second electrodes 322 arranged in the circumferential direction of the inner tube 2 is greater than the diameter of the first electrodes 321, which is not specifically limited here.

It should be understandable that in order to improve the ablation efficiency, the circumferentially distributed fourth electrodes, fifth electrodes and the like can be further arranged on the base bodies, which will not be illustrated in detail herein.

In an embodiment, the electrophysiology catheter further includes wires. The electrophysiology catheter is provided with a power source (a high voltage generator) at the tail end. The wires are connected with the electrodes and extend to the tail end to be electrically connected with the power source for the purpose of enabling the electrodes 32 to form positive and negative electrodes to form a field, so as to achieve the ablation to the lesion position.

Optionally, the wires can be disposed between the support rod and the insulation layer. Or, they can be disposed within the cavity of the tubular structure of the support rod. The wires have good electrical conductivity and can withstand a voltage of more than 1000V without being broken down.

Various embodiments of the present disclosure have been described above. The above description is exemplary but not exhaustive, and the present disclosure is not limited to the disclosed embodiments. Without deviating from the scope and spirit of the various embodiments illustrated, many modifications and changes are obvious to a person skilled in the art. The selection of terms used in this text aims to best explain the principles, the practical applications of various embodiments, or the improvements to the technologies in the market, or to enable other ordinary technical persons in the art to understand the disclosed embodiments.

## Claims

1. An electrophysiology catheter, **characterized in that** the electrophysiology catheter comprises an inner tube and an outer tube connected in a sleeving mode, wherein the inner tube and the outer tube are movably relative to each other, and the inner tube and the outer tube are connected with each other by an electrode assembly which comprises:
a plurality groups of base body groups which are arranged in a circumferential direction of the inner tube, wherein the base body group connects the inner tube with the outer tube and comprises two or more base bodies distributed at intervals in the circumferential direction of the inner tube, the base body is deformable and at least comprises a first segment and a second segment connected with each other, the first segment and the second segment have a first connecting point, the first connecting points of a same base body group are in contact connection with each other to form a junction; and
electrodes which are at least disposed at the junctions.

2. The electrophysiology catheter according to claim 1, **characterized in that** the electrode assembly at least has a first state and a second state, and the relative movement between the inner tube and the outer tube can drive the connected electrode assembly to switch between the first state and the second state,
wherein in the first state, the base body is attached to an outer wall of the inner tube in the circumferential direction of the inner tube, and an extension direction of the base body is parallel to an axis direction of the inner tube;
wherein in the second state, the base body bendingly deform to outwardly bulge relative to the inner tube, each adjacent first segments of a same base body group enclose to form a first closed shape and each adjacent second segments enclose to form a second closed shape, and each adjacent base body groups enclose to form a third closed shape.

3. The electrophysiology catheter according to claim 2, **characterized in that** in the second state, the plurality groups of base body groups circumferentially distributed around the inner tube have a largest diameter, and the junctions are located at one side of a plane where the largest diameter is located away from the outer tube.

4. The electrophysiology catheter according to claim 3, **characterized in that** the electrodes at least comprise first electrodes and second electrodes, wherein the first electrodes are located at the junctions, the second electrodes are disposed at the second segments, and the second electrodes are located at the positions with the largest diameter, or the second electrodes are located between the positions with the largest diameter and the junctions.

5. The electrophysiology catheter according to claim 4, **characterized in that** the first electrodes and the second electrodes are electrode rings in an annular structure, and following the circumferential direction of the inner tube, a circumferential area of the first electrodes is greater than a circumferential area of the second electrodes.

6. The electrophysiology catheter according to claim 4, **characterized in that**, along the axis direction of the inner tube, the first electrodes circumferentially arranged have a first diameter, and the second electrodes circumferentially arranged have a second diameter which is greater than the first diameter.

7. The electrophysiology catheter according to claim 4, **characterized in that** the electrode assembly comprises a plurality of first base seats which are circumferentially arranged on the inner tube and configured to connect the plurality groups of base body groups with the inner tube, and the base bodies of a same base body group are connected to different said first base seats respectively.

8. The electrophysiology catheter according to claim 4, **characterized in that** the electrode assembly comprises a plurality of second base seats which are circumferentially arranged between the inner tube and the outer tube at one side near the outer tube and configured to connect the plurality groups of base body groups with the outer tube, and the base bodies of a same base body group are connected to different said second base seats respectively.

9. The electrophysiology catheter according to claim 7, **characterized in that** the second base seat and the second segment have a second connecting point at a connecting position, the base body group comprises two base bodies, and the second connecting points at the connecting positions of adjacent base body groups with the same second base seat are in contact fit with each;
wherein the electrodes further comprise third electrodes, and the third electrode is located at the second connecting points which are in contact fit with each other.

10. The electrophysiology catheter according to claim 9, **characterized in that** the electrode assembly has a third state which is formed by a relative movement between the inner tube and the outer tube on the basis of the second state;
wherein in the third state, the third electrodes are circumferentially arranged in interspaces between the base body groups, the electrode assembly forms an umbrella structure, and along the axis direction of the inner tube the plane defined by the third electrodes is at least between the first electrodes and the second electrodes.

11. The electrophysiology catheter according to claim 9, **characterized in that** the electrode assembly has a fourth state which is formed by the relative movement between the inner tube and the outer tube on the basis of the second state;
wherein in the fourth state, the third electrodes are circumferentially arranged in the interspaces between the base body groups and at least the first electrodes, the second electrodes and the third electrodes are in a same plane.

12. The electrophysiology catheter according to any one of claims 1 to 11, **characterized in that** the base body comprises a support rod and an insulating layer, the support rod is a metal member with deformation ability, and the insulting layer covers a surface of the metal member.

13. The electrophysiology catheter according to claim 1, **characterized in that** the electrophysiology catheter has a head end and a tail end in an extension direction thereof, the electrode assembly is located at the head end, and the tail end is provided with an adjusting part which connects the sleeved inner tube and the outer tube and can control and drive the inner tube to move relative to the outer tube in the axis direction of the catheter.

14. The electrophysiology catheter according to claim 13, **characterized in that** the adjusting part comprises a button and a sliding component slidably connecting the inner tube with the outer tube.

15. The electrophysiology catheter according to claim 13, **characterized in that** the electrophysiology catheter further comprises wires, the electrophysiology catheter is provided with a power source at the tail end, and the wires are connected with the electrodes and extend to the tail end to be electrically connected with the power source.
